# EUROPEAN PATENT SPECIFICATION

(11) **EP 3 815 673 B1**
(45) Date of publication and mention of the grant of the patent: **07.09.2022**
(21) Application number: 19206396.4
(22) Date of filing: 31.10.2019
(51) Int. Cl.: A61K 8/49, A61Q 5/08, A61Q 5/10, A61K 8/22

(54) **TWO-PART BLEACHING COMPOSITION**
ZWEITEILIGE BLEICHMITTELZUSAMMENSETZUNG
COMPOSITION DE DÉCOLORATION EN DEUX PARTIES

(43) Date of publication of application: 05.05.2021
(73) Proprietor: Kao Germany GmbH, 64297 Darmstadt (DE)
(72) Inventor: BREAKSPEAR, Steven, 64297 Darmstadt (DE); BAUER, Peter, 64297 Darmstadt (DE); HEILMANN, Jens, 64297 Darmstadt (DE); BAUER, Diana, 64297 Darmstadt (DE)

(56) References cited:
- EP-A2- 2 559 456
- WO-A1-2009/053180
- DE-A1-102006 061 830
- DE-A1-102007 041 493

## Description

### Field of the invention

The present invention is directed to a two-part bleaching composition, a method for bleaching of keratin fibers, and a kit-of-parts for bleaching.

### Background of the invention

Aqueous, hydrogen peroxide-containing compositions are an essential component for effectively bleaching keratin fibers. In particular cosmetic industry relies on the performance of these compositions. Typically, hydrogen-peroxide compositions are formulated as emulsions.

However, a common problem of such emulsion compositions is that storage stability especially under suboptimal temperature conditions is dissatisfactory. For example, consumers living in countries closer to the equatorial line can very often not guarantee proper storage of the compositions at room temperature. As a result, the user is confronted with lower performance of bleaching processes compared to results obtained a couple of months ago.

However, it is essential for the user of hydrogen-peroxide emulsion compositions that they have a long shelf life in order to retain their original performance.

WO2009/053180 discloses aqueous oxidizing compositions comprising 5% by weight or less of hydrogen peroxide and purine derivatives, in particular caffeine. It was found that purine derivatives reduce damage to keratin fibers.

### Summary of the invention

Thus, the first object of the present invention is a two-part bleaching composition comprising:
- a bleaching powder composition A comprising one or more persalt(s) and/or peroxy salt(s) and one or more alkalizing agent(s),
- an aqueous oxidizing composition B having a pH in the range of 1 to 6 comprising:
   a) hydrogen peroxide, and
   b) one or more compound(s) according to the following structure: 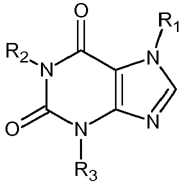 wherein R₁, R₂, and R₃ are independently selected from H and CH₃, and/or their mixtures, and
   c) one or more lipophilic compound,
wherein composition B is an oil-in-water emulsion.

The second object of the present invention is a method for bleaching and/or lightening of keratin fibers, preferably human keratin fibers, more preferably human hair comprising the steps of:
i) providing the two-part composition as defined above and mixing the compositions to provide a ready-to-use composition having a pH in the range of 7 to 12,
ii) applying the ready-to-use composition onto keratin fibers and leaving it for a time period in the range of 1 to 60 min,
iii) rinsing off the keratin fibers and optionally shampooing the keratin fibers.

A third object of the present invention is a kit-of-parts comprising the two-part composition as defined above and an optional aqueous composition having a pH in the range of 7 to 12 and comprising one or more oxidative dye precursors and/or oxidative dye couplers.

### Detailed description of the invention

Despite the attempts of the prior art, no satisfactory solution could be delivered to maintain performance and storage stability of hydrogen peroxide emulsion compositions.

Inventors of the present invention have unexpectedly found out that xanthine derivatives increase storage stability of hydrogen peroxide compositions at room, lowered, and elevated temperature. Moreover, xanthine derivatives enable the maintenance of composition viscosity over storage. This ensures mixability of the two-part composition and avoids handling problems for customers. Moreover, bleaching processes were found to be more consistent in performance over time by the inventive principle.

### Bleaching powder composition A

The bleaching powder composition A of the two-part bleaching composition comprises one or more persalt(s) and/or peroxy salt(s). Suitable persalts and/or peroxy salts are sodium persulfate, potassium persulfate, ammonium persulfate, earth alkali peroxides such as magnesium peroxide, melamine peroxide or urea peroxide or phthalimidoperoxy hexanoic acid. The preferred persalts from the viewpoint of bleaching power are sodium, potassium, and ammonium persulfate.

It is preferred from the viewpoint of bleaching power and cosmetic safety that the total concentration of persalts and/or peroxy salts in the bleach powder composition

A before mixing is in the range of 10% to 80% by weight, preferably 15% to 70% by weight, more preferably 20% to 60% by weight, and still more preferably 25% to 60% by weight, calculated to the total weight of the bleach powder composition A before mixing.

The bleaching powder composition A further comprises one or more alkalizing agent(s). Suitable alkalizing agent(s) are metasilicates, in particular sodium metasilicate. It is preferred from the viewpoint of alkalinity that the concentration of metasilicates in the bleaching powder composition A before mixing is in the range of 1% to 20% by weight, more preferably 5% to 15% by weight, calculated to the total weight of the bleaching powder composition A before mixing.

Other suitable alkalizing agent(s) are carbonate and bicarbonate alkali salts such as sodium, potassium, and ammonium salts. The preferred salts are bicarbonate salts and especially preferred is ammonium bicarbonate, from the viewpoint of buffer capacity. Suitable concentration of carbonates in the bleaching powder composition A is in the range of 0.25% to 10% by weight, preferably 0.5% to 7.5% by weight, more preferably 0.75% to 5% by weight, and still more preferably 1% to 4% by weight, calculated to the total weight of the bleaching powder composition A before mixing, from the viewpoint of buffer capacity and low hair damage.

Optionally, the bleaching powder composition A may comprise one or more ammonium salt(s) different from persalt(s) and peroxy salt(s).

Suitable ammonium salts different from persalt(s) and peroxy salt(s) are ammonium carbonate, ammonium hydrogen carbonate, ammonium carbamate, ammonium chloride, ammonium sulfate, ammonium phosphates, ammonium nitrate, ammonium bromide, ammonium iodide, ammonium thiosulfate, ammonium molybdate, ammonium vanadate, ammonium sulfamate, ammonium citrate, ammonium salicylate, ammonium valerate, ammonium tartarate, ammonium benzoate, ammonium acetate, ammonium formiate and ammonium lactate. Compositions may also comprise mixtures of ammonium salts.

The bleaching powder composition A may comprise one or more ammonium salts different from persalt(s) and peroxy salt(s) at a total concentration in the range of 0.1% to 10% by weight, calculated to the total weight of bleaching powder composition A.

Preferably, the bleaching powder composition A comprises less than 5% of water, more preferably it is an anhydrous composition, from the viewpoint of stability and maintaining a free-flowing powder. The term anhydrous is to be understood that no water is added to the powder. However, this does not exclude any water bound to the ingredients by, for example, capillary forces.

It is further preferred from the viewpoint of cosmetic safety that the bleach powder composition A is dust-free. This property can commonly be achieved by adding lipophilic compounds to the bleaching powder, preferably at concentration ranges from 1% to 10% by weight, calculated to the total weight of the bleach powder composition A.

### Aqueous oxidizing composition B

### Aqueous oxidizing composition

The aqueous oxidizing composition B has a pH in the range of 1 to 6 and comprises:
a) hydrogen peroxide, and
b) one or more compound(s) according to the following structure: 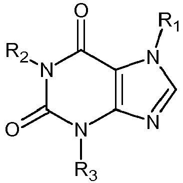 wherein R₁, R₂, and R₃ are independently selected from H and CH₃, and/or their mixtures, and
c) one or more lipophilic compound,
   wherein composition B is an oil-in-water emulsion.

It is preferred from the viewpoint of storage stability and cosmetic safety of the composition that the pH is 1.25 or more, more preferably 1.5 or more, further more preferably 2 or more.

It is preferred from the viewpoint of storage stability of the composition that the pH of the composition is 5 or less, more preferably 4 or less, further more preferably 3 or less.

For attaining the above-mentioned effects, it is preferred that the pH of the composition is in the range of 1.25 to 5, more preferably in the range of 1.5 to 4, further more preferably in the range of 2 to 3.

It is further preferred from the viewpoint of product performance that the concentration of the compound according to a) is in the range of 1% by weight or more, more preferably 2% by weight or more, further more preferably 3% by weight or more, calculated to the total weight of the composition.

It is further preferred from the viewpoint of product performance and user safety that the concentration of the compound according to a) is 20% by weight or less, more preferably 15% by weight or less, further more preferably 12% by weight or less, calculated to the total weight of the composition.

For attaining the above-mentioned effects, it is preferred that the concentration of the compound according to a) is in the range of 1% to 20% by weight, more preferably 2% to 15% by weight, further more preferably 3% to 12% by weight, calculated to the total weight of the composition.

Suitable xanthine and/or xanthine derivatives according to compound b) are
- Xanthine with R₁ = R₂ = R₃ = H,
- Theobromine with R₁ = R₃ = CH₃ and R₂ = H,
- Theophylline with R₂ = R₃ = CH₃ and R₁ = H, and
- Caffeine with R₁ = R₂ = R₃ = CH₃.

Mixtures of the above are suitable as well.

It is preferred from economic viewpoint that at least one compound according to b) is caffeine.

It is further preferred from the viewpoint of stabilizing performance that the total concentration of compounds according to b) is 0.001% by weight or more, more preferably 0.01% by weight or more, further more preferably 0.02% by weight or more, still more preferably 0.03% by weight or more, calculated to the total weight of the composition.

It is further preferred from the viewpoint of economic reasons as well as stabilizing performance that the total concentration of compounds according to b) is 0.5% by weight or less, more preferably 0.25% by weight or less, further more preferably 0.1% by weight or less, still more preferably 0.08% by weight or less, calculated to the total weight of the composition.

For attaining the above-mentioned effects it is preferred that the total concentration of compounds according to b) is in the range of 0.001% to 0.5% by weight, preferably 0.01% to 0.25% by weight, more preferably 0.02% to 0.1% by weight, still more preferably 0.03% to 0.08% by weight, calculated to the total weight of the composition.

The aqueous oxidizing composition B is an oil-in-water emulsion and comprises one or more lipophilic compound(s) as compound according to c).

Preferably, compounds according to c) are selected from C₁₂ to C₂₂ fatty alcohols, esters of C₃ to C₂₂ alcohols with C₁₂ to C₂₂ fatty acids, C₈ to C₂₂ fatty acids, vegetable oils, and/or silicones, and hydrocarbon-based products, and/or their mixtures, from the viewpoint of cosmetic compatibility.

Suitable C₁₂ to C₂₂ fatty alcohols are are myristyl alcohol, cetyl alcohol, stearyl alcohol, behenyl alcohol, and cetearyl alcohol.

Suitable esters of C₃ to C₂₂ alcohols with C₁₂ to C₂₂ fatty acids are isopropyl myristate, isopropyl palitate, and myristyl myristate.

Suitable C₈ to C₂₂ fatty acids are oleic acid, linoleic acid, and palmitic acid.

Suitable vegetable oils are olive oil, almond oil, sunflower oil, and argan oil.

Suitable silicones are non-aminated and/or aminated silicones. The latter are commonly known as amodimethicones.

Suitable hydrocarbon-based products are mineral oil, paraffins, and Vaseline.

It is preferred from the viewpoint of forming a stable composition and user friendliness that the total concentration of compounds according to c) is 1% by weight or more, more preferably 2% by weight or more, further more preferably 3% by weight or more, calculated to the total weight of the composition.

It is preferred from the viewpoint of forming a stable composition that the total concentration of compounds according to c) is 20% by weight or less, more preferably 15% by weight or less, further more preferably 12% by weight or less, calculated to the total weight of the composition.

For attaining the above-mentioned effects, the total concentration of compounds according to c) is in the range of 1% to 20% by weight, preferably 2% to 15% by weight, more preferably 3% to 12% by weight, calculated to the total weight of the composition.

### Surfactants as compounds according to d)

The bleach powder composition A and/or the aqueous oxidizing composition B of the present invention may further comprise one or more surfactant(s) as compound(s) according to d), preferably selected from non-ionic surfactants, anionic surfactants, cationic surfactants, and/or amphoteric/zwitterionic surfactants, and/or their mixtures, more preferably selected from anionic surfactants and/or non-ionic surfactants, from the viewpoint of stabilizing the composition and improving wettability and mixability.

Preferably, the anionic surfactants may be selected from ethoxylated or non-ethoxylated alkyl ether sulfate surfactants, alkyl sulfates, ethoxylated and/or non-ethoxylated alkyl carboxylates, ethoxylated or non-ethoxylated amino acid surfactants, and/or their mixtures.

Suitable examples are alkyl sulfate or preferably ethoxylated alkyl ether sulfate surfactant or mixtures thereof having an alkyl chain length of C₁₀ to C₂₂.

Suitable non-ionic surfactants may be selected from alkyl polyglycosides, ethoxylated triglycerides, ethoxylated fatty alcohols, ethoxylated fatty acid esters, and/or their mixtures.

Suitable cationic surfactants are quaternary ammonium surfactants having a carbon chain length in the range of C₁₂ to C₂₂ or surfactants having a tertiary amine group and at least one alkyl chain having a carbon chain length in the range of C₁₂ to C₂₂ such as alkylamidoalkylamine surfactants. Suitable examples are cetrimonium chloride.

Suitable amphoteric/zwitterionic surfactants are of betaine type. Suitable compounds may be selected from alkyl betaines and/or alkylamido betaines. A preferred compound selected from alkyl betaines is lauryl betaine. A preferred compound selected from alkylamido betaines is cocamidopropyl betaine. The disclosure also relates to the salts of the compounds.

It is preferred from the viewpoint of enhancing wettability of keratin fibers and mixability with other compositions as well as emulsion stability that the total concentration of compound(s) according to d) is in the range of 0.1% to 10% by weight, preferably 0.25% to 8% by weight, more preferably in the range of 0.5% to 5% by weight, calculated to the total weight of the compositions A and/or B.

### Viscosity of aqueous oxidizing composition B

The viscosity of the aqueous oxidizing composition B may be adjusted by emulsion components to achieve a cosmetically safe viscosity.

It is preferred from the viewpoint of cosmetic safety and mixability that the aqueous oxidizing composition B composition of the present invention has viscosity in the range of 1,000 mPas to 25,000 mPas, preferably 2,000 mPas to 20,000 mPas, more preferably in the range of 2,500 mPas to 17,500 mPas, determined by cone plate viscometry at 25°C under atmospheric conditions. A suitable viscometer is a Brookfield viscometer with spindle #4.

### Thickening polymers

In case the viscosity needs to be further adjusted, the bleach powder composition A and/or the aqueous oxidizing composition B may comprise one or more thickening polymers, from the viewpoint of cosmetic safety.

The compositions of the present invention may comprise one or more thickening polymer(s) selected from non-ionic thickening polymers and/or anionic thickening polymers, and/or their mixtures.

Preferably, the thickening polymers are selected from polymers resulting in an aqueous solution and/or aqueous dispersion at pH between 1 and 6 having a viscosity of at least 1,000 mPa•s measured at a polymer concentration of 1% by weight in water at 25°C, calculated to the total weight of the composition, determined by a Brookfield viscometer, such as at 10 rpm for 1 min, with an appropriate spindle at 25°C.

Suitable non-ionic thickening polymers are cellulose-based polymers. Suitable examples of cellulose-based polymers are methylcellulose, hydroxymethylcellulose, hydroxyethylcellulose, hydroxypropylcellulose, hydroxyethyl-methylcellulose, and alkylated hydroxyl celluloses such as (C₂-C₈)-alkylcelluloses or cetyl hydroxyethylcellulose.

Suitable anionic thickening polymers are selected from naturally-based anionic polymers and/or synthetic anionic polymers.

Suitably, the natural anionic polymer(s) may be selected from xanthan gum, dehydroxanthan gum, hydroxypropylxanthan gum, carboxymethyl cellulose and starch based polymers such as vegetable starch and/or their synthetically modified derivatives such as hydroxypropyl starch phosphate. Equally suitable are alginic acids, sodium alginates, ammonium alginates, calcium alginates, gum arabic, and guar gum.

The preferred thickening polymer for the composition of the present invention are natural anionic polymers, more preferably xanthan gum and/or dehydroxanthan gum, from the viewpoint of their biodegradability and low environmental impact.

Preferably, the total concentration of thickening polymers in the compositions A and/or B of present invention are 0.1% by weight or more, more preferably 0.25% by weight or more, more preferably 0.5% by weight or more, calculated to the total weight of the compositions A and/or B, from the viewpoint of providing sufficient viscosity to the composition.

Preferably, the total concentration of thickening polymers in the compositions A and/or B of present invention are 15% by weight or less, more preferably 12% by weight or more, further more preferably 10% by weight or less, calculated to the total weight of the compositions A and/or B, from the viewpoint of providing sufficient viscosity to the composition and cost of goods.

For attaining the above-mentioned effects, it is preferred that the total concentration of thickening polymers in the compositions A and/or B of present invention is in the range of 0.1% to 15% by weight, preferably 0.25% to 12% by weight, more preferably in the range of 0.5% to 10% by weight, calculated to the total weight of the compositions A and/or B.

### Ready-to-use mixture and method for bleaching and/or lightening

The present invention is also directed to a method for bleaching and/or lightening of keratin fibers, preferably human keratin fibers, more preferably human hair comprising the steps of:
i) providing the two-part composition as defined above and mixing the compositions to provide a ready-to-use composition having a pH in the range of 7 to 12,
ii) applying the ready-to-use composition onto keratin fibers and leaving it for a time period in the range of 1 to 60 min,
iii) rinsing off the keratin fibers and optionally shampooing the keratin fibers.

The bleach powder composition A is mixed with the aqueous oxidizing composition of B to form a ready-to-use composition. Suitable mixing ratios by weight are 5:1 to 1:5 (bleach powder composition A: aqueous oxidizing composition B). Customarily, suitable mixing ratios are 1:1, 1:2, and 1:3 by weight (bleach powder composition A: aqueous oxidizing composition B).

Suitably, the pH of the ready-to-use composition is in the range of 7 to 12. It is preferred from the viewpoint of accelerated bleaching that the pH of the ready-to-use composition is in the range of 7.5 to 11, more preferably 8.0 to 10.5.

The ready-to-use composition is then applied to keratin fibers and left for a time period of 1 min to 60 min as defined in step ii). Preferred time ranges for step ii) are 5 min to 45 min, more preferred ranges are 10 min to 35 min, from the viewpoint of sufficiently bleaching the keratin fibers.

After that, the ready-to-use composition is rinsed-off from keratin fibers and optionally they are shampooed and optionally blow-dried.

### Kit-of-parts

The present invention is also directed to a kit-of-parts comprising the two-part composition as defined above and an optional aqueous composition having a pH in the range of 7 to 12 and comprising one or more oxidative dye precursors and/or oxidative dye couplers.

Such kits are advantageous as three-part bleaching/dyeing compositions.

The following examples are to illustrate the present invention, but not to limit it.

### EXAMPLES

### Example 1

The following bleaching powder composition A was prepared:

| | % **by weight** |
|---|---|
| Hydroxyethylcellulose | 3 |
| Tetrasodium EDTA | 2 |
| Sodium carbonate | 1 |
| Ammonium persulfate | 11 |
| Potassium persulfate | 36 |
| Sodium metasilicate | 10 |
| Mineral oil | 8 |
| Diatomaceous Earth | ad 100.0 |

The following aqueous oxidizing compositions B were prepared by dispersing cetearyl alcohol and Ceteareth-30 in water and heating the mixture under constant stirring to 60°C. After cooling, the mixture is then added to hydrogen peroxide solution under constant stirring. Then caffeine was dissolved as compound according to b):

| **Ingredient** | **Inventive composition** | **Comparative composition** |
|---|---|---|
| | **1 [% by weight]** | **1 [% by weight]** |
| Caffeine | 0.03 | - |
| Hydrogen peroxide | 9.0 | |
| Cetearyl alcohol | 4.0 | |
| Ceteareth-30 | 0.5 | |
| Phosphoric acid | q.s. ad pH 2.0 | |
| Water | Ad 100.0 | |

The compositions were prepared and stored under controlled conditions at several temperatures for 1 month. Viscosity was measured and the results were found as follows:

| **Time [months]** | **Inventive composition 1 [mPas]** | | | | **Comparative composition 1 [mPas]** | | | |
|---|---|---|---|---|---|---|---|---|
| | **Storage Temperature** | | | | | | | |
| | **5°C** | **25°C** | **40°C** | **50°C** | **5°C** | **25°C** | **40°C** | **50°C** |
| 0 | 7,360 | | | | 8,360 | | | |
| 1 | 12,800 | 11,400 | 13,920 | 14,080 | 45,000 | 14,400 | 50,600 | 49,400 |

A further visual inspection of the compositions was performed and it was confirmed by the human operator that comparative composition 1 was foamy and presented gas bubble at the surface, whereas inventive composition 1 did not present any bubbles nor was it foamy.

As a result of the viscosity test, the viscosity of the inventive composition 1 increased by about 50% over 1 month, whereas the viscosity of the comparative composition increased over 500-600% under certain storage conditions.

The bleaching powder composition A from above was then mixed with the inventive composition 1 and comparative composition 1 both stored after 1 month at 40°C at a weight ratio of 1:2 (bleaching powder:inventive composition 1) to yield a ready-to-use composition having a pH around 9.8.

Due to the high viscosity of comparative composition 1, proper mixing with the bleaching powder composition was not possible leaving visible powder aggregates in the ready-to-use mixture. For complete dissolution, the mixture had to be further diluted with composition B.

The ready-to-use compositions were then applied onto human hair and left for 30 min at room temperature. After that, the compositions were rinsed-off with water, the hair was shampooed, and blow-dried.

The hair was found to be intensely bleached with inventive composition 1, whereas the degree of lightening was visually much lower for the hair streak bleached with comparative composition 1.

### Example 2

### Composition B

| | % **by weight** |
|---|---|
| Caffeine | 0.01 |
| Cetearyl alcohol | 4.0 |
| Sodium lauryl sulfate | 0.8 |
| Phosphoric acid | q.s. ad pH 1.5 |
| Tetrasodium EDTA | 0.05 |
| Light mineral oil | 3.0 |
| Hydrogen peroxide | 3.0 |
| Water | ad 100.0 |

The concentration of caffeine as compound according to b) may also be adjusted to 0.05%, 0.1%, or 0.5% or any values in between to achieve the same technical effect.

The concentration of hydrogen peroxide may be adjusted to 1%, 9%, 12%, 15%, or 20% by weight, or any value in between.

### Example 3

### Composition B

| | % **by weight** |
|---|---|
| Theobromine | 0.01 |
| Cetearyl alcohol | 4.0 |
| Sodium lauryl sulfate | 1.5 |
| Phosphoric acid | q.s. ad pH 2.5 |
| Tetrasodium EDTA | 0.05 |
| Sunflower oil | 3.0 |
| Stearyl alcohol | 3.0 |
| Hydrogen peroxide | 6.0 |
| Water | ad 100.0 |

The concentration of theobromine as compound according to b) may also be adjusted to 0.05%, 0.1%, or 0.5% or any values in between to achieve the same technical effect.

Theobromine may also be replaced by xanthin or theophylline.

The concentration of hydrogen peroxide may be adjusted to 3%, 9%, 12%, 15%, or 20% by weight, or any value in between.

### Example 4

### Composition B

| | % **by weight** |
|---|---|
| Caffeine | 0.01 |
| Theobromine | 0.05 |
| Theophylline | 0.05 |
| Cetearyl alcohol | 4.0 |
| Sodium lauryl sulfate | 0.8 |
| Phosphoric acid | q.s. ad pH 3.0 |
| Tetrasodium EDTA | 0.05 |
| Isopropyl myristate | 3.0 |
| Dimethicone 100 cs | 0.5 |
| Hydrogen peroxide | 3.0 |
| Water | ad 100.0 |

The concentration of theobromine as compound according to b) may also be adjusted to 0.05%, 0.1%, or 0.5% or any values in between to achieve the same technical effect.

Theobromine may also be replaced by xanthin or theophylline.

The concentration of hydrogen peroxide may be adjusted to 1%, 3%, 9%, 12%, 15%, or 20% by weight, or any value in between.

## Claims

1. A two-part bleaching composition comprising:
- a bleaching powder composition A comprising one or more persalt(s) and/or peroxy salt(s) and one or more alkalizing agent(s),
- an aqueous oxidizing composition B having a pH in the range of 1 to 6 comprising:
a) hydrogen peroxide, and
b) one or more compound(s) according to the following structure: 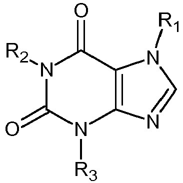 wherein R₁, R₂, and R₃ are independently selected from H and CH₃, and/or their mixtures, and
c) one or more lipophilic compound,
wherein composition B is an oil-in-water emulsion.

2. The composition according to claim 1 **characterized in that** the composition A comprises one or more alkalizing agent(s) at a total concentration in the range of 0.25% to 30% by weight, preferably 0.5% to 25% by weight, more preferably 1% to 20% by weight, calculated to the total weight of composition A.

3. The composition according to claims 1 and/or 2 **characterized in that** the composition A comprises one or more persalt(s) and/or peroxy salt(s) at a total concentration in the range of 10% to 80% by weight, preferably 15% to 70% by weight, more preferably 20% to 60% by weight, still more preferably 25% to 60% by weight, calculated to the total weight of composition A.

4. The composition according to any of the preceding claims **characterized in that** composition A comprises less than 5% of water, preferably it is an anhydrous composition.

5. The composition according to any of the preceding claims **characterized in that** composition A comprises one or more ammonium salt different from persalt(s) and peroxy salt(s).

6. The composition according to any of the preceding claims **characterized in that** the concentration of compound according to a) in composition B is in the range of 1% to 20% by weight, preferably 2% to 15% by weight, more preferably 3% to 12% by weight, calculated to the total weight of the composition B.

7. The composition according to any of the preceding claims **characterized in that** at least one compound according to b) is caffeine.

8. The composition according to any of the preceding claims **characterized in that** the total concentration of compounds according to b) in composition B is in the range of 0.001% to 0.5% by weight, preferably 0.01% to 0.25% by weight, more preferably 0.02% to 0.1% by weight, still more preferably 0.03% to 0.08% by weight, calculated to the total weight of composition B.

9. The composition according to any of the preceding claims **characterized in that** the compound according to c) is selected from C₁₂ to C₂₂ fatty alcohols, esters of C₃ to C₁₂ alcohols with C₁₂ to C₂₂ fatty acids, C₁₂ to C₂₂ fatty acids, vegetable oils, and/or silicones, and/or hydrocarbon-based products, and/or their mixtures.

10. The composition according to any of the preceding claims **characterized in that** the concentration of compounds according to c) is in the range of 1% to 20% by weight, preferably 2% to 15% by weight, more preferably 3% to 12% by weight, calculated to the total weight of composition B.

11. The composition according to any of the preceding claims **characterized in that** compositions A and/or B comprise one or more surfactant(s) as compound according to d), preferably selected from non-ionic surfactants, anionic surfactants, cationic surfactants, and/or amphoteric/zwitterionic surfactants, and/or their mixtures, more preferably selected from anionic surfactants.

12. The composition according to any of the preceding claims **characterized in that** the total concentration of compound(s) according to d) is in the range of 0.1% to 10% by weight, preferably 0.25% to 8% by weight, more preferably in the range of 0.5% to 5% by weight, calculated to the total weight of the compositions A and/or B.

13. The composition according to any of the preceding claims **characterized in that** the viscosity of composition B is in the range of 1,000 mPas to 25,000 mPas, preferably 2,000 mPas to 20,000 mPas, more preferably in the range of 2,500 mPas to 17,500 mPas, determined by cone plate viscometry at 25°C under atmospheric conditions.

14. A method for bleaching and/or lightening of keratin fibers, preferably human keratin fibers, more preferably human hair comprising the steps of:
i) providing the two-part composition as defined in claims 1 to 12 and mixing the compositions to provide a ready-to-use composition having a pH in the range of 7 to 12,
ii) applying the ready-to-use composition onto keratin fibers and leaving it for a time period in the range of 1 to 60 min,
iii) rinsing off the keratin fibers and optionally shampooing the keratin fibers.

15. Kit-of-parts comprising the two-part composition as defined in the claims 1 to 12 and an optional aqueous composition having a pH in the range of 7 to 12 and comprising one or more oxidative dye precursors and/or oxidative dye couplers.

## Patentansprüche

1. Zweiteilige Bleichzusammensetzung, aufweisend:
- eine Bleichpulverzusammensetzung A, welche ein oder mehrere Persalze und/oder Peroxysalze und ein oder mehrere Alkalisierungsmittel aufweist,
- eine wässrige oxidierende Zusammensetzung B mit einem pH-Wert im Bereich von 1 bis 6, aufweisend:
a) Wasserstoffperoxid, und
b) eine oder mehrere Verbindungen gemäß der folgenden Struktur: 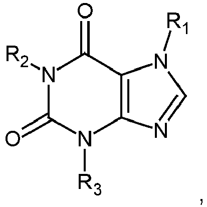 wobei R1, R2 und R3 unabhängig aus H und CH₃ und/oder deren Gemischen ausgewählt sind, und
c) eine oder mehrere lipophile Verbindungen, wobei die Zusammensetzung B eine ÖI-in-Wasser-Emulsion ist.

2. Zusammensetzung nach Anspruch 1, **dadurch gekennzeichnet, dass** die Zusammensetzung A ein oder mehrere Alkalisierungsmittel in einer Gesamtkonzentration im Bereich von 0,25 Gewichts-% bis 30 Gewichts-%, vorzugsweise 0,5 Gewichts-% bis 25 Gewichts-%, insbesondere 1 Gewichts-% bis 20 Gewichts-%, aufweist, bezogen auf das Gesamtgewicht der Zusammensetzung A.

3. Zusammensetzung nach Anspruch 1 und/oder 2, **dadurch gekennzeichnet, dass** die Zusammensetzung A ein oder mehrere Persalze und/oder Peroxysalze in einer Gesamtkonzentration im Bereich von 10 Gewichts-% bis 80 Gewichts-%, vorzugsweise 15 Gewichts-% bis 70 Gewichts-%, insbesondere 20 Gewichts-% bis 60 Gewichts-%, noch mehr bevorzugt 25 Gewichts-% bis 60 Gewichts-%, aufweist, bezogen auf das Gesamtgewicht der Zusammensetzung A.

4. Zusammensetzung nach einem der vorhergehenden Ansprüche, **dadurch gekennzeichnet, dass** die Zusammensetzung A weniger als 5 % Wasser aufweist, vorzugsweise ist sie eine wasserfreie Zusammensetzung.

5. Zusammensetzung nach einem der vorhergehenden Ansprüche, **dadurch gekennzeichnet, dass** die Zusammensetzung A ein oder mehrere Ammoniumsalze aufweist, die andere als die Persalze und die Peroxysalze sind.

6. Zusammensetzung nach einem der vorhergehenden Ansprüche, **dadurch gekennzeichnet, dass** die Konzentration an Verbindung gemäß a) in der Zusammensetzung B im Bereich von 1 Gewichts-% bis 20 Gewichts-%, vorzugsweise 2 Gewichts-% bis 15 Gewichts-%, insbesondere 3 Gewichts-% bis 12 Gewichts-%, liegt, bezogen auf das Gesamtgewicht der Zusammensetzung B.

7. Zusammensetzung nach einem der vorhergehenden Ansprüche, **dadurch gekennzeichnet, dass** mindestens eine Verbindung gemäß b) Koffein ist.

8. Zusammensetzung nach einem der vorhergehenden Ansprüche, **dadurch gekennzeichnet, dass** die Gesamtkonzentration an Verbindungen gemäß b) in der Zusammensetzung B im Bereich von 0,001 Gewichts-% bis 0,5 Gewichts-%, vorzugsweise 0,01 Gewichts-% bis 0,25 Gewichts-%, insbesondere 0,02 Gewichts-% bis 0,1 Gewichts-%, noch mehr bevorzugt 0,03 Gewichts-% bis 0,08 Gewichts-%, liegt, bezogen auf das Gesamtgewicht der Zusammensetzung B.

9. Zusammensetzung nach einem der vorhergehenden Ansprüche, **dadurch gekennzeichnet, dass** die Verbindung gemäß c) aus C₁₂-C₂₂-Fettalkoholen, Estern von C₃-C₁₂-Alkoholen mit C₁₂-C₂₂-Fettsäuren, C₁₂-C₂₂-Fettsäuren, pflanzlichen Ölen und/oder Silikonen und/oder Produkten auf Kohlenwasserstoffbasis und/oder deren Gemischen ausgewählt ist.

10. Zusammensetzung nach einem der vorhergehenden Ansprüche, **dadurch gekennzeichnet, dass** die Konzentration an Verbindungen gemäß c) im Bereich von 1 Gewichts-% bis 20 Gewichts-%, vorzugsweise 2 Gewichts-% bis 15 Gewichts-%, insbesondere 3 Gewichts-% bis 12 Gewichts-%, liegt, bezogen auf das Gesamtgewicht der Zusammensetzung B.

11. Zusammensetzung nach einem der vorhergehenden Ansprüche, **dadurch gekennzeichnet, dass** die Zusammensetzungen A und/oder B ein oder mehrere Tenside als Verbindung gemäß d) aufweisen, vorzugsweise ausgewählt aus nichtionischen Tensiden, anionischen Tensiden, kationischen Tensiden und/oder amphoteren/zwitterionischen Tensiden und/oder deren Gemischen, insbesondere ausgewählt aus anionischen Tensiden.

12. Zusammensetzung nach einem der vorhergehenden Ansprüche, **dadurch gekennzeichnet, dass** die Gesamtkonzentration an Verbindung(en) gemäß d) im Bereich von 0,1 Gewichts-% bis 10 Gewichts-%, vorzugsweise 0,25 Gewichts-% bis 8 Gewichts-%, insbesondere im Bereich von 0,5 Gewichts-% bis 5 Gewichts-%, liegt, bezogen auf das Gesamtgewicht der Zusammensetzungen A und/oder B.

13. Zusammensetzung nach einem der vorhergehenden Ansprüche, **dadurch gekennzeichnet, dass** die Viskosität der Zusammensetzung B im Bereich von 1.000 mPas bis 25.000 mPas, vorzugsweise 2.000 mPas bis 20.000 mPas, insbesondere im Bereich von 2.500 mPas bis 17.500 mPas, liegt, bestimmt durch Kegel-Platte-Viskosimetrie bei 25 °C unter atmosphärischen Bedingungen.

14. Verfahren zum Bleichen und/oder Aufhellen von Keratinfasern, vorzugsweise menschlichen Keratinfasern, insbesondere menschlichen Haaren, umfassend die Schritte:
i) Bereitstellen der zweiteiligen Zusammensetzung nach Anspruch 1 bis 12 und Vermischen der Zusammensetzungen, um eine gebrauchsfertige Zusammensetzung bereitzustellen, welche einen pH-Wert im Bereich von 7 bis 12 aufweist,
ii) Aufbringen der gebrauchsfertigen Zusammensetzung auf Keratinfasern und Belassen derselben dort für eine Dauer im Bereich von 1 min bis 60 min,
iii) Ausspülen der Keratinfasern und gegebenenfalls Shampoonieren der Keratinfasern.

15. Mehrteiliges Kit, welches die zweiteilige Zusammensetzung nach Anspruch 1 bis 12 und eine optionale wässrige Zusammensetzung aufweist, welche einen pH-Wert im Bereich von 7 bis 12 aufweist und eine oder mehrere Oxidativfarbstoff-Vorstufen und/oder Oxidativfarbstoff-Kupplungsmittel aufweist.

## Revendications

1. Composition de décoloration en deux parties comprenant :
- une composition de décoloration A en poudre comprenant un ou plusieurs persels et/ou sels peroxy et un ou plusieurs agents alcalinisants,
- une composition oxydante aqueuse B ayant un pH dans la plage de 1 à 6 comprenant :
a) du peroxyde d'hydrogène, et
b) un ou plusieurs composés selon la structure suivante : 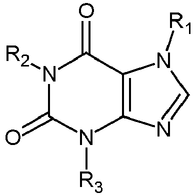 où R₁, R₂, et R₃ sont choisis de manière indépendante parmi un atome H et un groupement CH₃, et/ou leurs mélanges, et
c) un ou plusieurs composés lipophiles,
où la composition B est une émulsion huile dans eau.

2. Composition selon la revendication 1, **caractérisée en ce que** la composition A comprend un ou plusieurs agents d'alcalinisation dans une concentration totale dans la plage de 0,25 % à 30 % en poids, de préférence de 0,5 % à 25 % en poids, plus préférentiellement de 1 % à 20 % en poids, calculée par rapport au poids total de la composition A.

3. Composition selon les revendications 1 et/ou 2, **caractérisée en ce que** la composition A comprend un ou plusieurs persels et/ou sels peroxy à une concentration totale dans la plage de 10 % à 80 % en poids, de préférence de 15 % à 70 % en poids, plus préférentiellement de 20 % à 60 % en poids, plus préférentiellement encore de 25 % à 60 % en poids, calculée par rapport au poids total de la composition A.

4. Composition selon l'une quelconque des revendications précédentes, **caractérisée en ce que** la composition A comprend moins de 5 % d'eau, c'est de préférence une composition anhydre.

5. Composition selon l'une quelconque des revendications précédentes, **caractérisée en ce que** la composition A comprend un ou plusieurs sels d'ammonium différents de persels ou de sels peroxy.

6. Composition selon l'une quelconque des revendications précédentes, **caractérisée en ce que** la concentration d'un composé selon a) dans la composition B est dans la plage de 1 % à 20 % en poids, de préférence de 2 % à 15 % en poids, plus préférentiellement de 3 % à 12 % en poids, calculée par rapport au poids total de la composition B.

7. Composition selon l'une quelconque des revendications précédentes, **caractérisée en ce qu'**au moins un composé selon b) est la caféine.

8. Composition selon l'une quelconque des revendications précédentes, **caractérisée en ce que** la concentration totale de composés selon b) dans la composition B est dans la plage de 0,001 % à 0,5 % en poids, de préférence de 0,01 % à 0,25 % en poids, plus préférentiellement de 0,02 % à 0,1 % en poids, plus préférentiellement encore de 0,03 % à 0,08 % en poids, calculée par rapport au poids total de la composition B.

9. Composition selon l'une quelconque des revendications précédentes, **caractérisée en ce que** le composé selon c) est choisi parmi des alcools gras en C₁₂ à C₂₂, des esters d'alcools en C₃ à C₁₂ avec des acides gras en C₁₂ à C₂₂, des acides gras en C₁₂ à C₂₂, des huiles végétales et/ou silicones, et/ou des produits à base d'hydrocarbures, et/ou leurs mélanges.

10. Composition selon l'une quelconque des revendications précédentes, **caractérisée en ce que** la concentration de composés selon c) est dans la plage de 1 % à 20 % en poids, de préférence de 2 % à 15 % en poids, plus préférentiellement de 3 % à 12 % en poids, calculée par rapport au poids total de la composition B.

11. Composition selon l'une quelconque des revendications précédentes, **caractérisée en ce que** les compositions A et/ou B comprennent un ou plusieurs tensioactifs servant de composés selon d), de préférence choisis parmi des tensioactifs non ioniques, des tensioactifs anioniques, des tensioactifs cationiques, des tensioactifs amphotères/zwitterioniques, et/ou leurs mélanges, plus préférentiellement choisis parmi des tensioactifs anioniques.

12. Composition selon l'une quelconque des revendications précédentes, **caractérisée en ce que** la concentration totale de composés selon d) est dans la plage de 0,1 % à 10 % en poids, de préférence de 0,25 % à 8 % en poids, plus préférentiellement dans la plage de 0,5 % à 5% en poids, calculée par rapport au poids total des compositions A et/ou B.

13. Composition selon l'une quelconque des revendications précédentes, **caractérisée en ce que** la viscosité de la composition B est dans la plage de 1 000 mPas à 25 000 mPas, de préférence de 2 000 mPas à 20 000 mPas, plus préférentiellement dans la plage de 2 500 mPas à 17 500 mPas, déterminée par un viscosimètre cône plateau à 25 °C dans des conditions atmosphériques.

14. Procédé de décoloration et/ou d'éclaircissement de fibres de kératine, de préférence de fibres de kératine humaine, plus préférentiellement de cheveux humains, comprenant les étapes :
i) de mise au point de la composition en deux parties selon les revendications 1 à 12 et de mélange des compositions afin de fournir une composition prête à l'emploi ayant un pH dans la plage de 7 à 12,
ii) d'application de la composition prête à l'emploi sur des fibres de kératine et de maintien pendant un intervalle de temps dans la plage de 1 à 60 min,
iii) de rinçage des fibres de kératine et de shampouinage éventuel des fibres de kératine.

15. Kit en parties comprenant la composition en deux parties selon les revendications 1 à 12 et une composition aqueuse éventuelle ayant un pH dans la plage de 7 à 12, et comprenant un ou plusieurs précurseurs de coloration oxydatifs et/ou des coupleurs de coloration oxydatifs.
